# EUROPEAN PATENT SPECIFICATION

(11) **EP 2 844 243 B1**
(45) Date of publication and mention of the grant of the patent: **13.11.2019**
(21) Application number: 13724007.3
(22) Date of filing: 03.05.2013
(51) Int. Cl.: A61K 31/35, A61K 36/185, A61K 31/352, C07D 311/80

(54) **METHOD FOR PREPARING A CANNABIS PLANT ISOLATE COMPRISING DELTA-9-TETRAHYDROCANNABINOL**
VERFAHREN ZUR HERSTELLUNG EINES CANNABIS-PFLANZENISOLATES MIT DELTA-9-TETRAHYDROCANNABINOL
PROCÉDÉ DE PRÉPARATION D'UN ISOLAT DE PLANTES DE CANNABIS COMPRENANT DU DELTA-9-TETRAHYDROCANNABINOL

(30) Priority: 03.05.2012 EP 12166661
(43) Date of publication of application: 11.03.2015
(73) Proprietor: Echo Pharmaceuticals B.V., 1382GS Weesp (NL)
(72) Inventor: FERNANDEZ CID, Maria Vanesa, NL-2012 KE Haarlem (NL); VAN HOUTEN, Dennis, NL-1525 PV Westknollendam (NL)
(74) Representative: Nederlandsch Octrooibureau
(86) International application number: PCT/NL2013/050339
(87) International publication number: WO 2013/165251

(56) References cited:
- WO-A1-00/25127
- WO-A2-03/064407
- WO-A2-2004/026857
- GB-A- 2 408 978

## Description

### TECHNICAL FIELD OF THE INVENTION

The present invention relates to a method for preparing a Cannabis plant Δ9-tetrahydrocannabinol isolate from a crude solvent extract of Cannabis plant material.

### BACKGROUND OF THE INVENTION

Since many years cannabis has been used as a medicament for use in the treatment of various diseases and disorders. The interest in the pharmacology of cannabis goes back hundreds of years. In addition to uses as anasthetics, spasmolytics and hypnotics, cannabinoids have been used to combat emesis and nausea induced by cancer chemotherapy, and also in the treatment of glaucoma. In recent times, cannabinoids have been looked at with sepsis due to its abuse potential. A considerable part of the synthetic effort has been directed toward the preparation of some of the oxygenated human urinary metabolites of Δ9-tetrahydrocannabinol for use in forensic science as analytical standards for the detection of marijuana use.

Several developments have contributed to the current renewed interest of pharma companies in this area. The indentification of cannabinoid receptors (CB1 and CB2) in rat brain (Devane et al., Mol. Pharmacol., 34:605-613; 1988) was a considerable step forwards. The involvement of the pharmaceutical industry in this area has resulted in a more specific knowledge about the chemistry, and pharmacokinetic- and pharmacodynamic properties of cannabinoids.

Cannabinoids, which are substituted meroterpenes are the major active constituents of the plant Cannabis sativa. The most important natural cannabinoid is the psychoactive tetrahydrocannabinol (Δ9-tetrahydrocannabinol; hereinafter THC); others include the non-psychoactive (but pharmaceutically active) compounds cannabidiol (hereinafter: CBD) and cannabigerol (hereinafter CBG).

Cannabinoids can be administered by a variety of routes. Because of their high lipid solubility, topical administration is possible in locations such as for example the eye or the nose. However, this has been of very limited applicability.

Oral administration results in a slow and variable absorption with a bio-availability of 10-20%, usually less than 15%. Intravenous injection or infusion is possible, but because of the very low water-solubility of cannabinoids a special formulation must be used, such as a complex of the cannabinoid with plasma protein, or a solution in a water-miscible organic solvent. Intravenous administration of suitable preparations gives a very rapid onset of action, but because of dosage limitations to avoid excessive intensity of the peak effect, the duration of action is relatively short.

Smoking has been the most commonly used method of administration, and is the typical manner of using crude marijuana, as opposed to pure cannabinoids. Much of the total THC in crude cannabis is not free THC but tetrahydrocannabinolic acid. The heat just ahead of the advancing zone of combustion in a cigarette or pipeful of cannabis converts the THC acid to free THC, and volatizes the THC so that it can be inhaled with the smoke, deep into the lungs. The high lipid-solubility of THC allows it to cross the alveolar membrane rapidly, entering the blood in the pulmonary capillaries, and allowing a fast uptake into the brain.

Although crude marijuana is often used by patients suffering from diseases and disorders for which cannabinoids provide relief, such crude products are less suitable for use in pharmaceutical formulations. For such applications use is preferably made of purified forms of certain cannabinoids, (e.g. THC) present in herbal cannabis. The methods known in the art for preparing such purified forms of cannabinoids are laborious and are not very efficient, i.e. they need relatively high amounts of cannabis plant material and use expensive and toxic solvents. This is particularly true for the preparation of cannabis extracts wherein the main constituent is THC.

WO2004/026857 provides a method for preparing a purified cannabis extract, wherein the cannabinoids are purified to at least 99% wt% THC (Δ-9- tetrahydrocannabinol). In this method a crude ethanolic extract of Cannabis plant material is passed through a column of activated charcoal and evaporated by means of rotary evaporation. The resulting THC enriched extract is subsequently passed through a column packed with Sephadex LH20 and eluted with chloroform/dichloromethane. The solvent used is removed by means of rotary evaporation. In order to further increase the purity of the THC enriched extract, the extract is dissolved in methanol and subsequently in pentane and subjected to rotary evaporation twice. Although this method provides a extract with at least 99 wt% THC, it is very laborious and uses for the chromatographic separation solvents, such as chloroform and dicloromethane, which are considered a health risk. Moreover, some solvent, such as methanol, may remain in the product obtained. Furthermore, due to the chromatography column used batch to batch variability with respect to the purity of the cannabinoid obtained will frequently occur. The yield of THC obtained with this method is also relatively low.

Hence, a need remains for a method for obtaining a high purity THC extract from Cannabis in good yield, which method is relatively simple to carry out and wherein no use is made of solvents which are considered a health risk, or wherein the solvents used are removed to such an extent that they do not pose a health risk. Furthermore, a need exists for a method that yields a well-defined extract in which the levels of active cannabinoids, e.g. THC, CBN and CBD vary within narrow boundaries. Such an isolate would be very useful as a pharmaceutically active component in pharmaceutical compositions.

### SUMMARY OF THE INVENTION

The inventors have developed an isolation method that meets the aforementioned *desiderata.* The method according to the present invention comprises:
a) providing a crude solvent extract of Cannabis plant material;
b) subjecting the crude extract to thin film evaporation to obtain a refined extract;
c) chromatographically fractionating the refined extract to produce one or more high purity fractions having a THC content higher than a preset value and one or more low purity fractions having a THC content lower than said preset value, wherein the preset value is in the range of 95-99% by weight of dry matter;
d) subjecting the one or more high purity fractions to another thin film evaporation; and
e) collecting a THC isolate containing at least 97% THC by weight of dry matter; and wherein in step b) and/or in step d) the thin film evaporation is carried out by using wiped film evaporation.

The isolation method of the present invention offers the advantage that it yields a high purity THC extract in good yield and without using solvents that pose a health risk. The method further offers the advantage that it is highly reproducible in that it produces THC-isolate with a specific cannabinoid profile. More particularly, the method yields a THC isolate that contains at least 97.0-99.5% THC and 0.4-2.0% of other cannaniboids, including at least 0.3% Cannabinol and Cannabidiol (all percentages by weight of dry matter).

### DEFINITIONS

The term "cannabinoid" or "cannabinoids" as used herein encompasses at least the following substances: Δ-8 tetrahydrocannabinol, Δ-9- tetrahydrocannabinol (THC), cannabinol (CBN), olivetol, cannabidiol (CBD), cannabigerol (CBG), Δ-9(11)-tetrahydrocannabinol (exo-THC), cannabichromene (CBC), tetrahydrocannabinol-C3 (THC-C3), tetrahydrocannabinol-C4 (THC-C4).

The term "cannabinoid acid", refers to the acid form of the above mentioned cannabinoids.

The term "Cannabis plant(s)" refers to wild type Cannabis sativa and also variants thereof, including cannabis chemovars (varieties characterized by means of chemical composition) which naturally contain different amounts of the individual cannabinoids, also *Cannabis sativa* subspecies indica including the variants *var. indica* and *var. kafiristanica*, Cannabis *indica* and also plants which are the result of genetic crosses, self-crosses or hybrids thereof.

The term "Cannabis plant material" encompasses a plant or plant part, e.g. leaves, stems, roots, flowers, seeds or parts thereof.

The term "crude solvent extract of Cannabis plant material" refers to an extract of Cannabis plant material, which extract comprises by weight of dry matter 20-90% THC, 0.1-2.0% CBN and 0.1-1.0% CBD.

The term "decarboxylation treatment" as used herein refers to a process step wherein Cannabis plant material has been treated such that the cannabinoid acids present in the untreated Cannabis plant material have been transformed into the corresponding free cannabinoids. Decarboxylation is usually carried out by heating the Cannabis plant material.

The term "winterization" as used herein refers to a process which involves the chilling of a solvent extract of Cannabis plant material below 0°C for removal of amongst others fats and waxes, optionally combined with filtration and/or centrifugation.

The term "thin film evaporation" as used herein has its normal scientific meaning and refers to a method of evaporation wherein the mixture to be evaporated flows down the (heated) walls of an evaporator as a film.

The term "wiped film evaporation" as used herein has its normal scientific meaning and refers to a method of evaporation wherein the mixture to be evaporated is spread by wiper blades on the (heated) walls of an evaporator as a film.

The term "flash chromatography" as used herein has its normal scientific meaning as described in amongst others Still et al., in J. Org. Chem. Vol. 43, No. 14., 1978.

### DETAILED DESCRIPTION OF THE INVENTION

A first aspect of the present invention relates to a method for preparing a Cannabis plant THC (Δ9-tetrahydrocannabinol) isolate from a crude solvent extract of Cannabis plant material, comprising:
a) providing a crude solvent extract of Cannabis plant material containing, by weight of dry matter, 20-90% Δ9-tetrahydrocannabinol (THC), 0.1-2.0.% Cannabinol (CBN) and 0.1.-1.0.% Cannabidiol (CBD);
b) subjecting the crude extract to thin film evaporation to obtain a refined extract;
c) chromatographically fractionating the refined extract to produce one or more high purity fractions having a THC content higher than a preset value and one or more low purity fractions having a THC content lower than said preset value, wherein the preset value is in the range of 95-99% by weight of dry matter;
d) subjecting the one or more high purity fractions to another thin film evaporation; and
e) collecting a THC isolate containing at least 97% THC by weight of dry matter; and wherein in step b) and/or in step d) the thin film evaporation is carried out by using wiped film evaporation, preferably both in step b) and in step d) use is made of wiped film evaporation

With the method of the present invention it is possible to prepare with a relatively high yield and in a few steps a Cannabis plant THC isolate which comprises a high amount of THC. Furthermore, with the present invention it is possible to use solvents which are not considered harmful to humans.

The crude solvent extract of Cannabis plant material that is used as a starting material in the present method contains, by weight of dry matter 20-90%, more preferably 20-70%, most preferably 20-40% THC.

The crude solvent extract is suitably prepared using a dried Cannabis extract that was obtained by extracting Cannabis flos with C5-C8 alkane, preferably hexane. Even more preferably, the crude solvent extract is prepared by extracting the latter dried Cannabis extract with a C1-C3 alcohol, especially methanol or ethanol.

The crude solvent extract typically contains 5-30 wt.%, more preferably 10-20 wt.% of a solvent. Examples of solvents that can be contained in the crude solvent extract include alcohols, C5-C8 alkane and combinations thereof. Preferably, the solvent is an alcohol, more preferably a C1-C3 alcohol, such as methanol or ethanol.

Preferably, the crude solvent extract of Cannabis plant material has been subjected to decarboxylation. Typically the crude solvent extract has been decarboxylated by heating it to a temperature of at least 100°C for at least 7 hours.

The crude solvent extract preferably has been subjected to a winterization treatment that comprised cooling of the crude solvent extract to a temperature below -10°C.

In a preferred embodiment of the present invention, the wiped film evaporator used is a short path wiped film evaporator..

During thin film evaporation step, in particular by using wiped film evaporation, as much solvent as possible, such as for example methanol, is evaporated from the crude solvent extract. Typically, the amount of solvent in the refined extract is reduced to less than 0.5 wt.%, even more preferably to less than 0.3 wt.% and most preferably to less than 0.03 wt.% in this thin film evaporation step.

The use of wiped film evaporation offers the advantage that residual solvent can be removed very efficiently.

According to a particularly preferred embodiment of the present method the crude extract is first subjected to rotary evaporation, i.e. until a relatively viscous composition is obtained, and subsequently to thin film evaporation, preferably wiped film evaporation, such that the refined extract is obtained.

Typically, the solvent content of the crude solvent extract is reduced to 1-15 wt.%, more preferably 1-10 wt.% and most preferably to 1-5 wt.% by rotary evaporation before it is subjected to thin film evaporation, preferably wiped film evaporation, to produce the refined extract.

In a further step, the refined extract is subjected to chromatographical fractionation, preferably flash chromatography. It is particularly preferred to use a mixture of C5-C8 alkane and ethylacetate as eluent for the chromatographical fractionation. Such a mixture comprises preferably 1-10 vol% ethylacetate, most preferably 5-10 vol% ethylacetate. According to a particularly preferred embodiment, the eluent employed is a mixture of C5-C6 alkane and ethyl acetate. Most preferably, the eluent is a mixture of hexane and ethyl acetate.

In order to further enhance the efficiency (e.g. yield) of the method according to the present invention the one or more low purity fractions produced in step c) of the method according to the present invention are combined with the crude solvent extract and/or the refined extract. Most preferably, the one or more low purity fractions are combined with the crude solvent extract.

According to the present invention, the high purity fraction obtained in step c) of the method of the present invention is subjected to a second or further thin film evaporation step, preferably wiped film evaporation. In this step the solvents used in step c) are substantially removed.

Typically, the amount of solvent contained in the one or more high purity fraction is reduced to less than 0.5 wt.%, even more preferably to less than 0.3. wt.% and most preferably to less than 0.03 wt.% in the thin film evaporation step, preferably wiped film evaporation step.

In order to further increase the efficiency of the present method it is preferred to first subject the one or more high purity fractions obtained from step c) to rotary evaporation and to subsequently subject these fractions to thin film evaporation, preferably wiped film evaporation.

According to a particularly preferred embodiment the solvent content of the crude solvent extract is reduced to 1-15 wt.%, more preferably 1-10 wt.% and most preferably to 1-5 wt.% by rotary evaporation before it is subjected to thin film evaporation, preferably wiped film evaporation, to produce the THC-isolate.

Preferably, the Cannabis plant THC isolate obtained by the present method comprises 97.2 - 98.0% THC by weight of dry matter. It is further preferred that such a THC isolate comprises at least 0.02% CBN by weight of dry matter.

In a preferred embodiment of the present invention the cannabis plant THC isolate obtained by the present method comprises at least 0.05% CBD by weight of dry matter.

It is further preferred that the Cannabis plant THC isolate obtained by the present method comprises at least 0.02% CBC by weight of dry matter.

In a preferred embodiment of the present invention the Cannabis plant THC isolate obtained by the present method comprises at least 0.02% THC-C3 by weight of dry matter.

Preferably, the Cannabis plant THC isolate obtained by the present method comprises at least 0.02% THC-C4 by weight of dry matter.

In a particularly preferred embodiment of the present invention, the components a) to j), as mentioned above, represent together at least 99.0% by weight of dry matter.

Preferably, the residual amount of methanol in the THC isolate obtained by the present method is below 10.000 ppm, more preferably below 5000 ppm. This way a product is obtained wherein the amount of methanol is so low that it does not pose a health risk.

The invention will now be illustrated by way of the following, non limiting examples.

### EXAMPLES

### Example 1

A Cannabis plant 9-tetrahydrocannabinol (THC) isolate according to the present invention was prepared as follows.

2 kg of Cannabis flos (i.e. the flowers) was obtained from Cannabis sativa plants and heated to about 100°C, in order to decarboxylate the cannabinoid acids present in the cannabis flos. The decarboxylated cannabis flos (1.8 kg) was subsequently mixed with hexane (ratio 1 g cannabis flos per 10 ml hexane) in order to extract the cannabinoids from the cannabis flos. The extract was separated from the flos and dried in a rotary evaporator to dryness. The solvent extract (about 600 g) comprised about 55% THC by dry weight.

Subsequently, the dried extract was brought into methanol (ratio 1 g dried extract per 2 ml methanol) and cooled to -10°C and subsequently filtered and centrifuged at 5000 RPM (Beckman Coulter, type Avanti J-26xp) for 25 min at 6°C. To the methanolic mixture obtained, active coal purity p.a. was added in a concentration of 10% by weight on dried extract. The mixture was filtrated and centrifuged again. The solvent extract (about 440 g) obtained comprised about 60% THC by weight of dry matter.

In order to remove the methanol from the mixture, the mixture was brought in a rotary evaporator (Rotavapor® Buchi R-210) and dried with the oil bath temperature of 60°C and rotation in position 5 until vacuum is below 25 mbar. The (partly) dried and viscous mixture with not more than 3 wt % of methanol was brought into a wiped film evaporator (UIC, type KDL-1) with an oil bath temperature of 180°C, water bath temperature of 70°C and a rotor speed of 100 RPM for 45 minutes and dried further such that a refined THC extract of about 380 g was obtained.

The refined THC extract was subsequently fractionated in a high purity fraction with a THC purity ≥ 98% and low purity fraction with a THC purity of ≤ 20% by means of flash chromatography (Büchi, Fraction Collector type C-660 and UV-detector type C-635) using an ethylacetate / hexane mixture (7% v/v ethylacetate in hexane) as eluent and a pre-packed column of normal silica, with an eluent flow of 250 ml/min, sample flow of 50 ml/min, detection wavelength of 275 nm for 30 min. The fractions were (partly) dried in a rotary evaporator (Rotavapor® Buchi R-210) with the oil bath temperature of 60°C and rotation in position 5 until vacuum is below 25 mbar. The high purity fraction of about 195 g was dried to dryness with a wiped film evaporator (UIC, type KDL-1) with an oil bath temperature of 180°C, water bath temperature of 70°C and a rotor speed of 100 RPM for 45 minutes and the THC isolate obtained of about 160 g was packed in glass syringes for further use.

Analysis of the cannabis plant THC isolate thus obtained has shown that the THC isolate comprises more than 98% THC, and about 0.13 % CBN, 0.3% CBD, 0% delta-8-THC, 0% exo-THC, 0% CBG, 0.1% CBC, 0.1% THC-C3 en 0.1% THC-C4 by weight of dry matter.

### Example 2

A tablet comprising the Cannabis plant 9-tetrahydrocannabinol (THC) isolate obtained with the method as described in Example 1 was prepared as follows.

Sucrose monolaurate (HLB=15) and Cannabis plant THC isolate were heated under a stream of nitrogen till 120°C. The THC to sucrose monolaurate ratio was 1:15 by weight. After thoroughly mixing, the putty-like melt was saturated with CO₂ (and thereby softened) following one of either method below:
- The warm melt was poured into a 120° C preheated autoclave and brought to 250 bars. The autoclave was pressurized with carbon dioxide using a plunger pump (LeWa) and heated by means of a jacket, using heating oil. The lump was further liquefied through saturation with CO₂ by stirring the melt in the supercritical CO₂ for al least 30 mins using a Buchi(TM) magnetic stirrer.
- The melt was chilled to -20°C and crushed to obtain maximum surface area. To this end a - 20°C pre-cooled mortar was used in an inert and dry atmosphere. The obtained powder was poured into a 60°C pre-warmed autoclave and brought to 250 bars. The autoclave was pressurized with carbon dioxide using a plunger pump (LeWa) and heated by means of a jacket, using heating oil. The vessel was further heated to 120°C with heating oil and hot CO2 (120°C) under continuous stirring allowing optimal CO₂-dissolvation.

After terminating the stirring, the melt was allowed to settle at the bottom of the autoclave. The valve at the bottom of the autoclave was opened. The high pressure in the autoclave forced the melt through a 120°C heat traced pipe into a 120°C -heat traced 340 µm nozzle (Spraying Systems Inc). Powder was formed upon depressurization from 250 bars to atmospheric pressure. The microgranulates had an average diameter of 30 µm as determined by light microscopy. A tabletting powder for direct compression was mixed using the following ingredients:
- 50 mg of the microgranulate
- 4 mg SiO2 (aerosol)
- 15 mg sodium starch glycolate (Primojel(TM)
- 60 mg NaHCO3
- 50 mg citric acid (1 aq.)

The powder was compressed applying a 15kN force to obtain a 10mm tablet with a total weight of 129 mg. The tablet strength was 4ON and the tablet disintegrated in 60 seconds in water of 37°C, forming a micro emulsion. The powder mixing and tabletting was performed in a dry and inert atmosphere.

### Comparative example

For comparison the method as already described in example 1 was substantially followed, however instead wiped film evaporation, use was made of rotary evaporation. As will be shown in the following, the difference in the product obtained is surprising and remarkable.

The 9-tetrahydrocannabinol (THC) isolate in this comparative was prepared as follows:

700 g of Cannabis flos (i.e. the flowers) was obtained from Cannabis sativa plants and heated to about 100°C, in order to decarboxylate the cannabinoid acids present in the cannabis flos. The decarboxylated cannabis flos (615 g) was subsequently mixed with hexane (ratio 1 g cannabis flos per 10 ml hexane) in order to extract the cannabinoids from the cannabis flos. The extract was separated from the flos and dried in a rotary evaporator to dryness. The solvent extract (about 250 g) comprised about 28% THC by dry weight.

Subsequently, the dried extract was brought into methanol (ratio 1 g dried extract per 2 ml methanol) and cooled to -100C and subsequently filtered and centrifuged at 5000 RPM (Beckman Coulter, type Avanti J-26xp) for 25 min at 60°C. To the methanolic mixture obtained, active coal purity p.a. was added in a concentration of 10% by weight on dried extract. The mixture was filtrated and centrifuged again. The solvent extract (about 170.3 g) obtained comprised about 30% THC by weight of dry matter.

Part of the THC extract obtained (about 40 g) was subsequently processed. In order to remove the methanol from the mixture, the mixture was brought in a rotary evaporator (Rotavapor® Buchi R-210) and dried with the oil bath temperature of 60°C and rotation in position 5 until vacuum was below 25 mbar.

The THC extract was subsequently fractionated in a high purity fraction with a THC purity ≥ 98% and low purity fraction with a THC purity of ≤ 20% by means of flash chromatography (Büchi, Fraction Collector type C-660 and UV-detector type C-635) using an ethylacetate / hexane mixture (7% v/v ethylacetate in hexane) as eluent and a pre-packed column of normal silica, with an eluent flow of 100 ml/min, sample flow of 50 ml/min, detection wavelength of 275 nm.

The high purity fraction was dried to dryness in a rotary evaporator (Rotavapor® Buchi R-210) with the oil bath temperature of 600C and rotation in position 5 until vacuum is below 25 mbar. The final product was 7.8 g.

The THC isolate obtained was analyzed and has shown that comprises by weight of dry matter:
a) 77% Δ9-tetrahydrocannabinol (THC);
b) 0.46% Cannabinol (CBN)
c) 0% Δ8-tetrahydrocannabinol (Δ8-THC);
d) 0% Δ9(11)-tetrahydrocannabinol (exo-THC)
e) 0% Cannabidiol (CBD)
f) 0.4% Cannabigerol (CBG)
g) 0.38% Cannabichromene (CBC)
h) 0% Tetrahydrocannabinol-C3 (THC-C3)
i) 0.22% Tetrahydrocannabinol-C4 (THC-C4)

Clearly, the THC isolate obtained has a significantly lower THC content as obtained with the method of the present invention, as exemplified in example 1. Furthermore, the THC isolate of the present invention comprised a considerably lower amount of methanol as the THC isolate obtained with the method of this comparative example. Moreover, the yield of from extract to final THC isolate was with the method of the present invention considerably higher than the yield obtained with the method of this comparative example. In this regard, reference is made to Table 1 below:

| | **Yield (%) from extract to final product** | **THC purity (% by weight)** | **Residual solvent** |
|---|---|---|---|
| | | | Methanol |
| With WFE (as in example1) | 36.4 % | > 98 % | ≤ 3000 ppm |
| With Rotary evaporator (as in comparative example) | 19.5 % | 77 % | 40751 ppm |

## Claims

1. A method for preparing a Cannabis plant Δ9-tetrahydrocannabinol (THC) isolate from a crude solvent extract of Cannabis plant material, comprising:
a) providing a crude solvent extract of Cannabis plant material containing, by weight of dry matter, 20-90% THC, 0.1-2.0 % Cannabinol (CBN) and 0.1-1.0 % Cannabidiol (CBD);
b) subjecting the crude extract to thin film evaporation to obtain a refined extract;
c) chromatographically fractionating the refined extract to produce one or more high purity fractions having a THC content higher than a preset value and one or more low purity fractions having a THC content lower than said preset value, wherein the preset value is in the range of 95-99% by weight of dry matter;
d) subjecting the one or more high purity fractions to another thin film evaporation; and
e) collecting a THC isolate containing at least 97% THC by weight of dry matter;
and wherein in step b) and/or in step d) the thin film evaporation is carried out by wiped film evaporation.

2. Method according to claim 1, wherein in step b) and in step d) the thin film evaporation is carried out by wiped film evaporation.

3. Method according to claim 1 or 2, wherein the wiped film evaporator used in step b) and/or d) is a short path wiped film evaporator.

4. Method according to any of the previous claims, wherein the chromatographical fractionation in step c) is carried out by flash chromatography.

5. Method according to claim 4, wherein the flash chromatography is carried out with a hexane / ethylacetate mixture as eluent, preferably the mixture comprises 1-10 vol% ehtylacetate.

6. Method according to any one of the previous claims, wherein one or more of the low purity fractions produced in step c) are combined with the crude solvent extract and/or the refined extract.

## Patentansprüche

1. Verfahren zur Herstellung eines Cannabis-Pflanze-Δ9-Tetrahydrocannabinol(THC)-Isolats aus einem rohen Lösungsmittelextrakt von Cannabis-Pflanzenmaterial, umfassend:
a) Bereitstellen eines rohen Lösungsmittelextrakts von Cannabis-Pflanzenmaterial, der 20-90% THC, 0,1-2,0% Cannabinol (CBN) und 0,1-1,0% Cannabidiol (CBD) enthält, bezogen auf das Gewicht der Trockenmasse;
b) Durchführung einer Dünnfilmverdampfung mit dem Rohextrakt, wobei man einen raffinierten Extrakt erhält;
c) chromatographisches Fraktionieren des raffinierten Extrakts unter Bildung von einer oder mehreren Fraktionen hoher Reinheit mit einem THC-Gehalt, der höher ist als ein voreingestellter Wert, und einer oder mehreren Fraktionen geringer Reinheit mit einem THC-Gehalt, der niedriger ist als der voreingestellte Wert, wobei der voreingestellte Wert im Bereich von 95-99 Gew.-% der Trockenmasse liegt;
d) Durchführung einer weiteren Dünnfilmverdampfung mit der einen oder den mehreren Fraktionen hoher Reinheit; und
e) Auffangen eines THC-Isolats, das wenigstens 97% THC enthält, bezogen auf das Gewicht der Trockenmasse;
wobei in Schritt b) und/oder in Schritt d) die Dünnfilmverdampfung durch Wischfilmverdampfung durchgeführt wird.

2. Verfahren gemäß Anspruch 1, wobei in Schritt b) und in Schritt d) die Dünnfilmverdampfung durch Wischfilmverdampfung durchgeführt wird.

3. Verfahren gemäß Anspruch 1 oder 2, wobei der in Schritt b) und/oder d) verwendete Wischfilmverdampfer ein Kurzweg-Wischfilmverdampfer ist.

4. Verfahren gemäß einem der vorstehenden Ansprüche, wobei die chromatographische Fraktionierung in Schritt c) durch Flash-Chromatographie durchgeführt wird.

5. Verfahren gemäß Anspruch 4, wobei die Flash-Chromatographie mit einem Hexan/Ethylacetat-Gemisch als Eluent durchgeführt wird, wobei vorzugsweise das Gemisch 1-10 Vol.-% Ethylacetat umfasst.

6. Verfahren gemäß einem der vorstehenden Ansprüche, wobei eine oder mehrere der in Schritt c) erzeugten Fraktionen geringer Reinheit mit dem rohen Lösungsmittelextrakt und/oder dem raffinierten Extrakt kombiniert werden.

## Revendications

1. Procédé de préparation d'un isolat de Δ9-tétrahydrocannabinol (THC) de plante de cannabis à partir d'un extrait brut de matière de plante de cannabis obtenu par solvant, comprenant :
a) prévoir un extrait brut de matière de plante de cannabis obtenu par solvant contenant, en poids de matière sèche, entre 20 et 90 % de THC, entre 0,1 et 2,0 % de cannabinol (CBN) et entre 0,1 et 1,0 % de cannabidiol (CBD) ;
b) soumettre l'extrait brut à une évaporation en couche mince pour obtenir un extrait raffiné ;
c) fractionner par chromatographie l'extrait raffiné pour produire une ou plusieurs fractions de pureté élevée présentant une teneur en THC supérieure à une valeur prédéterminée et une ou plusieurs fractions de faible pureté présentant une teneur en THC inférieure à ladite valeur prédéterminée, dans lequel la valeur prédéterminée est comprise entre 95 et 99 % en poids de matière sèche ;
d) soumettre les une ou plusieurs fractions de pureté élevée à une autre évaporation en couche mince ; et
e) recueillir un isolat de THC contenant au moins 97 % de THC en poids de matière sèche ;
et dans lequel, à l'étape b) et/ou à l'étape d), l'évaporation en couche mince est réalisée par une évaporation sur surface raclée.

2. Procédé selon la revendication 1, dans lequel, à l'étape b) et à l'étape d), l'évaporation en couche mince est réalisée par une évaporation sur surface raclée.

3. Procédé selon la revendication 1 ou 2, dans lequel l'évaporateur à surface raclée utilisé à l'étape b) et/ou d) est un évaporateur à surface raclée à court trajet.

4. Procédé selon l'une quelconque des revendications précédentes, dans lequel le fractionnement chromatographique de l'étape c) est réalisé par chromatographie flash.

5. Procédé selon la revendication 4, dans lequel la chromatographie flash est réalisée en utilisant un mélange d'hexane / acétate d'éthyle en tant qu'éluant, ce mélange comprenant de préférence entre 1 et 10 % en volume d'acétate d'éthyle.

6. Procédé selon l'une quelconque des revendications précédentes, dans lequel une ou plusieurs des fractions de faible pureté produites à l'étape c) sont combinées à l'extrait brut obtenu par solvant et/ou à l'extrait raffiné.
